# EUROPEAN PATENT SPECIFICATION

(11) **EP 2 352 533 B1**
(45) Date of publication and mention of the grant of the patent: **10.01.2018**
(21) Application number: 09830656.6
(22) Date of filing: 24.11.2009
(51) Int. Cl.: A61M 1/36, A61M 1/38, B01D 36/04, G01N 33/50, A61K 35/12

(54) **SYSTEM AND METHOD FOR SEPARATING NUCLEATED CELLS FROM BODY FLUIDS**
SYSTEM UND VERFAHREN ZUM TRENNEN VON NUKLEIERTEN ZELLEN AUS KÖRPERFLÜSSIGKEITEN
SYSTÈME ET PROCÉDÉ POUR SÉPARER DES CELLULES NUCLEÉS DE LIQUIDES BIOLOGIQUES

(30) Priority: 01.12.2008 US 325672; 12.12.2008 US 333926
(43) Date of publication of application: 10.08.2011
(73) Proprietor: General Electric Company, Schenectady, NY 12345 (US)
(72) Inventor: MILLER, Peter, New London Connecticut 06320 (US); POLIZZOTTI, Brian, D., Jamaica Plain Massachusetts 02130 (US); SMITH, Reginald, D., Niskayuna New York 12309 (US); SOOD, Anup, Niskayuna New York 12309 (US); WOOD, Nichole, L., New York 12309-1027 (US); YU, Liming, Piscataway New Jersey 08855 (US); ZHOU, Hongyi, New York 12309 (US)
(74) Representative: Aldenbäck, Ulla Christina
(86) International application number: PCT/SE2009/051327
(87) International publication number: WO 2010/064973

(56) References cited:
- WO-A1-93/25295
- WO-A1-96/29346
- WO-A1-2007/049286
- WO-A1-2007/049286
- WO-A2-2009/002849
- DE-A1-102004 018 347
- US-A- 4 663 058
- US-A- 5 482 829
- US-A- 5 670 060
- US-A- 6 027 688
- US-A1- 2003 134 416
- US-A1- 2003 134 416
- US-A1- 2007 083 145
- US-B1- 6 444 471
- DINO DI CARLO ET AL: "Equilibrium separation and filtration of particles using differential inertial focusing", ANALYTICAL CHEMISTRY, AMERICAN CHEMICAL SOCIETY, US, vol. 80, no. 6, 15 March 2008 (2008-03-15) , pages 2204-2211, XP002496651, ISSN: 0003-2700, DOI: 10.1021/AC702283M [retrieved on 2008-02-15]
- SUNGYOUNG CHOI ET AL: "Continuous blood cell separation by hydrophoretic filtration", LAB ON A CHIP, vol. 7, no. 11, 1 November 2007 (2007-11-01), pages 1532-1538, XP055068845, ISSN: 1473-0197, DOI: 10.1039/b705203k

## Description

### BACKGROUND

The invention relates generally methods for processing complex biological materials into subcomponents.

Separation of red blood cells (RBC) from whole blood is commonly required prior to analysis or therapeutic use of less abundant cells, such as white blood cells or stem cells. Many conventional blood cell isolation procedures require preliminary red blood cell depletion and sample volume reduction. These are commonly required processing steps for long-term cell banking and regenerative medicine applications where a maximal yield of rare cells is desired in a reduced volume due to storage limitations and/or the small volume requirements needed for direct transplantation. Today, the most common techniques for processing blood-cell containing samples (e.g. cord blood, bone marrow, peripheral blood) involve density-gradient sedimentation using centrifugation with or without the use of a density-gradient media to improve separations. Automated centrifugal systems have recently been developed for closed-system processing of cord blood and bone marrow samples in order to meet the growing needs for high-throughput sample processing. While greatly improving throughput compared to manual techniques, centrifugation-based devices have limited flexibility and portability due to the weight and fixed physical dimensions of the centrifuge bucket.

Filtration techniques are also used in a number of blood cell separation applications. For example, depth filtration has been used for sometime to achieve removal of leukocytes from whole blood (e.g. for transfusion applications). However these filters are designed for maximal leukocyte depletion (via trapping of cells within the filter) and have not been designed for high cell recovery following the filtration step. In addition, membrane-based plasmapheresis is a common technique for removal and processing of plasma from whole blood. However, these techniques do not involve pre-depletion of the whole blood of red blood cells (RBC) prior to filtration and do not achieve the type of volume reduction that is needed in blood cell banking applications.

Sedimentation methods, either via gravity or centrifugation, are known in the art for separating different components of blood. One method to facilitate sedimentation of RBCs from whole blood is to use polymeric large molecules, such as dextran, hetastarch, or gelatin, which are known aggregating agent for RBCs. Depending on the composition and stoichiometric ratio of the aggregating agent in blood, the speed and effectiveness of the RBC sedimentation process can vary widely. Some of the sedimentation-enhancing agents are known, such as potassium oxalate and potassium malonate. The effectiveness of these sedimentation-enhancing agents is largely determined by the concentration of the agent relative to the blood sample. Although potassium oxalate and malonate have previously been demonstrated as effective RBC sedimentation enhancing agents, the clinical utility of these agents is limited by the potential cardiovascular toxicity associated with potassium salt.

### BRIEF DESCRIPTION

The invention is adapted to address the need for a functionally high throughput method for processing biological materials, such as whole blood, while achieving high target cell (such as stem cell) recoveries and viabilities for downstream cell therapy applications. Filtration is a commonly used technique for blood processing application including hemodialysis and plasmaphersis but has not previously been used in blood cell banking applications where there is a need to process biological materials such as whole blood in order to remove red blood cells and excess plasma to achieve a concentrated white blood cell (WBC) sample. This is due to the challenges associated with separating abundant red blood cells from less abundant white blood cells and even less abundant stem cells of similar size. One of the embodiments of the methods comprises a two-step process involving an initial RBC aggregation and gravity sedimentation step for bulk erythrocyte removal, followed by a filtration step for cell concentration and removal of excess plasma.

US2003/134416 A1 describes a method and system for processing a blood sample and separate rare cell where sedimentation and filtration of blood cells is performed to collect rare cells. Dextran is used as aggregating agent, there is no description of an aggregation enhancing agent.

WO96/29346 A1 describes a method for producing a stable polymerized hemoglobin blood-substitute, including separating red blood cells from white blood cells including releasing hemoglobin to form a Hg solution treated by chromatography to for a Hg elute. The Hg elute is contacted with a crosslinking agent for forma polymerization reaction mixture (i.e. aggregating agent). There is no description of an aggregation enhancing agent.

An example of the methods for processing biological material generally comprises: providing a biological material in a vessel; adding an aggregating agent and an aggregation enhancing agent selected from citrate, succinate, a salt thereof, or a combination thereof to the material in the vessel and allowing the material to separate into two or more distinct submaterials; extracting one or more of the submaterials from the vessel; automatically transporting one or more of the submaterials remaining in the vessel to a filtration device via a conduit; and directing a resulting target retentateinto a target retentatereceptacle.

One example of the methods comprises processing blood samples for subsequent cryopreservation and/or direct therapeutic applications, e.g. to reduce sample volume, achieve high recovery and viability of nucleated cells, and remove the majority of red blood cells present in the starting sample.

One example of the methods enables one to isolate a white blood cell (WBC) ²fraction, which comprises pluripotent stem cells, from whole cord blood, bone marrow, or peripheral blood (including GCSF stimulated peripheral blood). At least one of the example methods of the invention is capable of achieving high leukocyte recoveries (>80%), >95% CD34 recovery, and high leukocytecell viabilities (>95%), while providing flexibility in handling a broad range of starting volumes and sample types based on adjustment of filtration times and filter cartridges used.

Unlike current methods, the methods of the invention enable automated processing of complex biological fluids without requiring users to purchase and use a separate centrifuge. The methods of the invention are also readily adaptable to handle a range of starting volumes, to concentrate a sample to a user-specified final volume, and for use in multiplexing processes (e.g. increasing/decreasing number of samples processed/run).

In general, the methods of the invention provide sedimentation-enhancing agents that are biocompatible and significantly increase the efficiency of blood separation methods and systems and thereby increase the recovery of total nucleated cells (TNC). At the concentration range specified, these sedimentation-enhancing agents are considered non-toxic and safe to use in vivo.

One or more examples of the method to sediment cells in a sample comprising blood cells comprises adding an aggregating agent; and a non-toxic enhancer having a final concentration range from about 10 mM to about 100 mM.

In some of the examples of the method to sediment cells is provided in a sample comprising blood cells comprises addition of an aggregating agent and the non-toxic enhancer comprises sodium citrate or sodium succinate or a combination thereof.

Some embodiments of the method to sediment cells improve the resulting recovery of an increased percentage of total nucleated cells from a sample comprising red blood cells, wherein the method comprises the steps of adding an aggregating agent, a non-toxic enhancer, incubating the sample to aggregate plurality of RBCs, and recovering the total nucleated cells.

### DRAWINGS

These and other features, aspects, and advantages of the present invention will become better understood when the following detailed description is read with reference to the accompanying drawings in which like characters represent like parts throughout the drawings, wherein:
FIG. 1 is a schematic drawing of an embodiment of the system for use in the method of the invention showing a biological sample and an RBC aggregating agent (w/ or w/o enhancer) in a mixing vessel and an agent receptacle, respectively.
FIG. 2 is a schematic drawing of the embodiment shown in FIG. 1 showing the aggregating agent (w or w/o enhancer) drawn into an extraction device.
FIG. 3 is a schematic drawing of the embodiment shown in FIG. 2 showing the aggregating agent (w or w/o enhancer) agent mixed into the biological sample in the vessel.
FIG. 4 is a schematic drawing of the embodiment shown in FIG. 3 showing a portion of the agent/sample mixture drawn into the extraction device.
FIG. 5 is a schematic drawing of the embodiment shown in FIG. 4 showing the drawn portion returned to the vessel.
FIG. 6 is a schematic drawing of the embodiment shown in FIG. 5 showing the mixture in a state of settling.

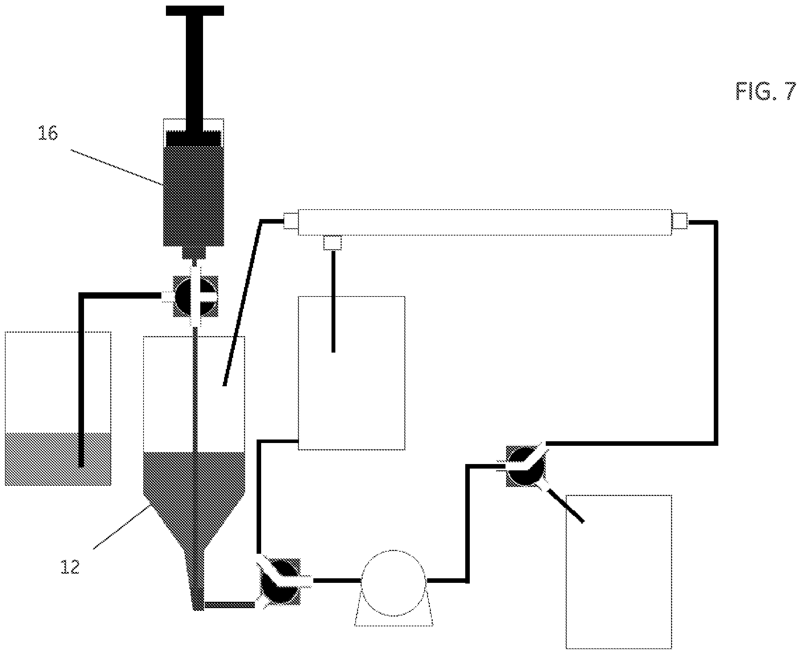

FIG. 7 is a schematic drawing of the embodiment shown in FIG. 6 showing the lowermost layer of the settled mixture drawn into the extraction device.

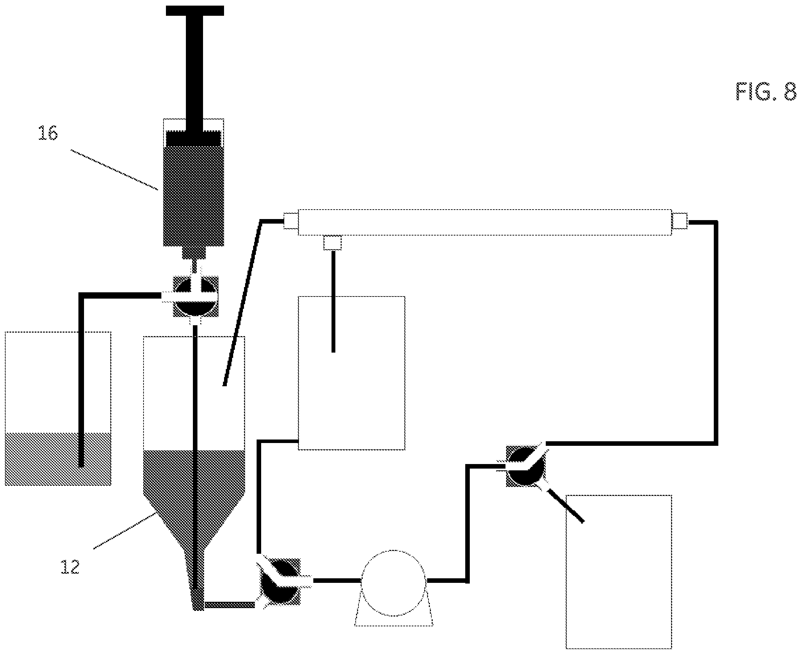

FIG. 8 is a schematic drawing of the embodiment shown in FIG. 7 showing a syringe valve between the extraction device and the vessel in a closed position.

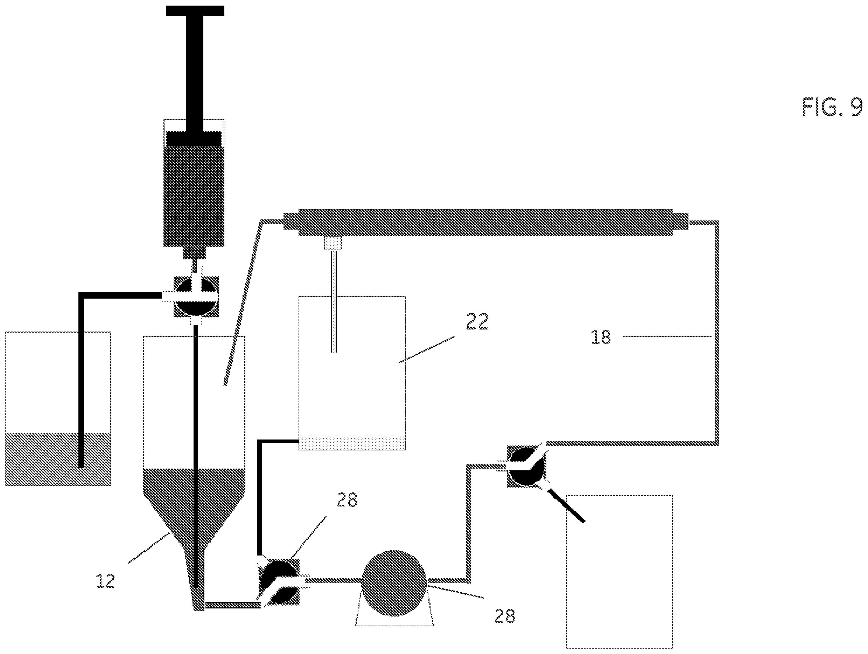

FIG. 9 is a schematic drawing of the embodiment shown in FIG. 8 showing a pump valve between the vessel and a pump in an open position and the mixture flowing through the system from the vessel through a conduit to a filtration device.

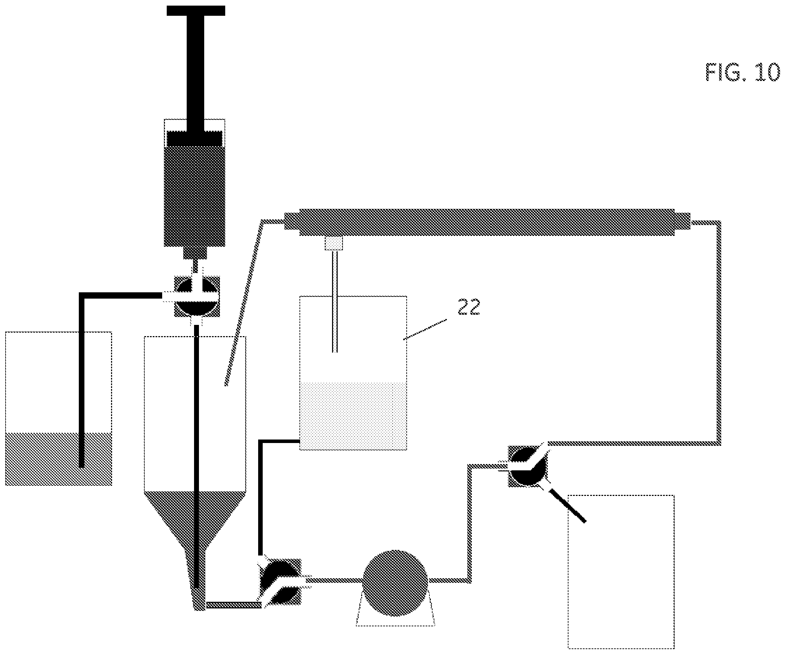

FIG. 10 is a schematic drawing of the embodiment shown in FIG. 9 showing the filter waste being collected in a waste filtration receptacle and the sample recirculating through the system.

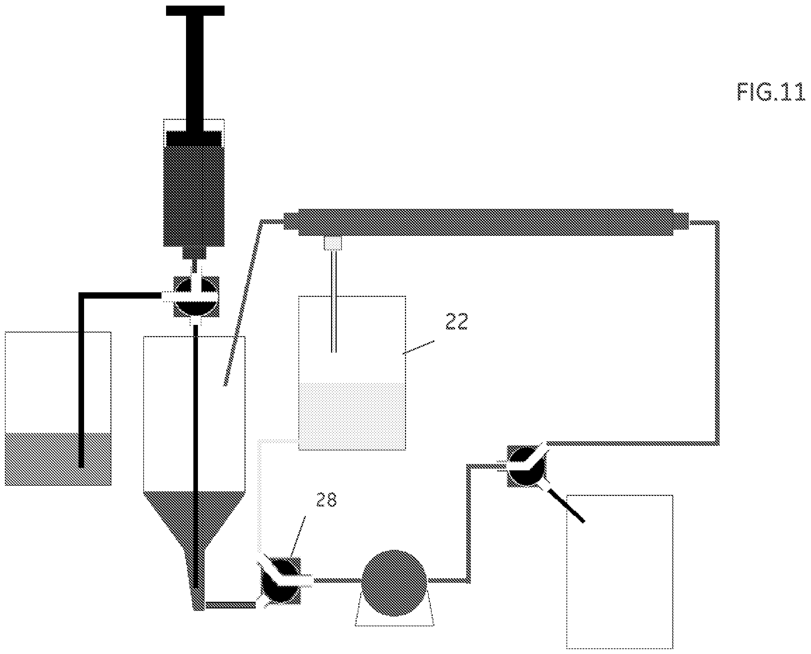

FIG. 11 is a schematic drawing of the embodiment shown in FIG. 10 showing the pump inlet valve in a closed position relative to the vessel and in an open position relative to the waste filtration receptacle and the waste filtrate recirculating through the conduit and filtration device.

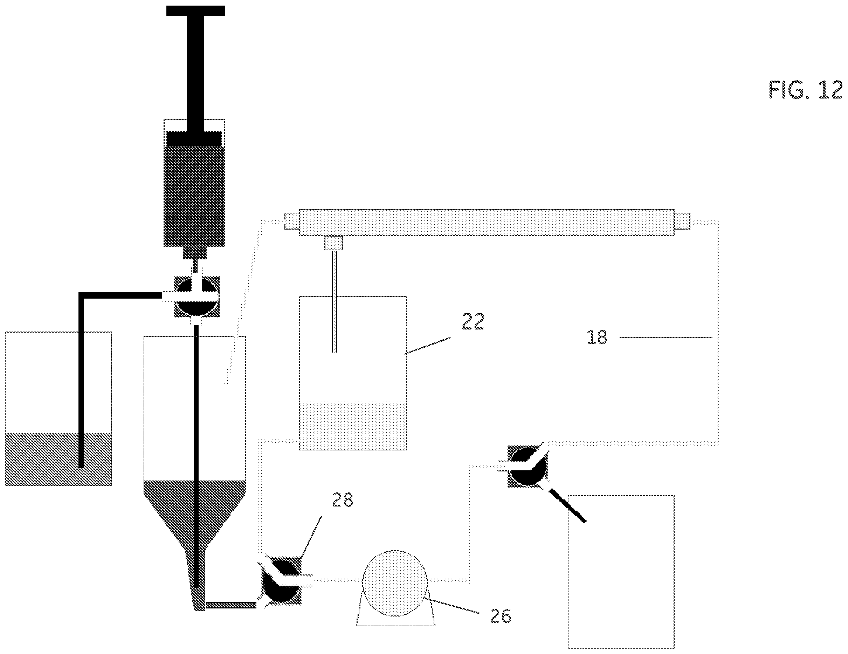

FIG. 12 is a schematic drawing of the embodiment shown in FIG. 11 showing the waste filtrate pumped through the system until it has replaced a target retentate trapped in the fluid path.

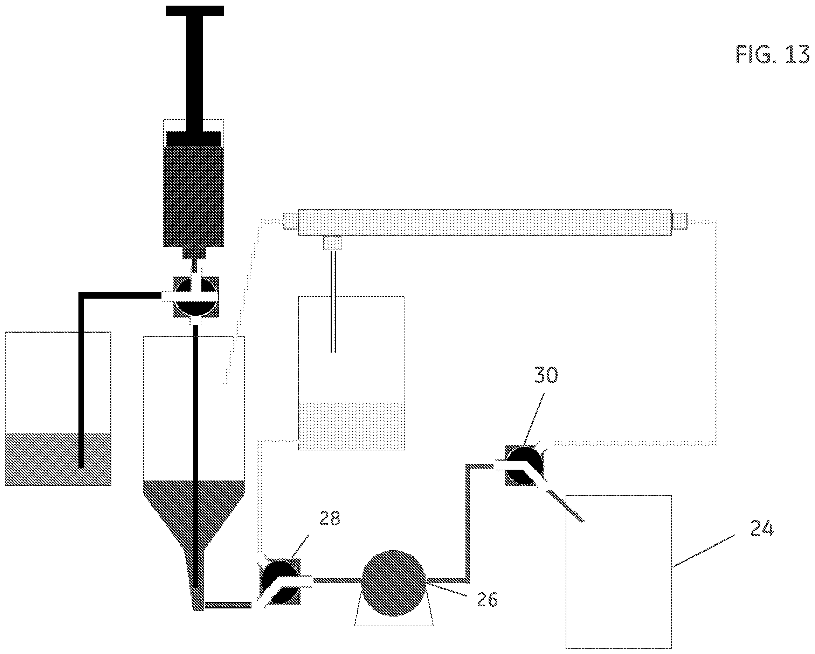

FIG. 13 is a schematic drawing of the embodiment shown in FIG. 12 showing the pump inlet valve in a closed position relative to the waste filtrate receptacle and in an open position relative to the vessel, and a pump outlet valve, between the pump and a target retentate receptacle, in an open position.

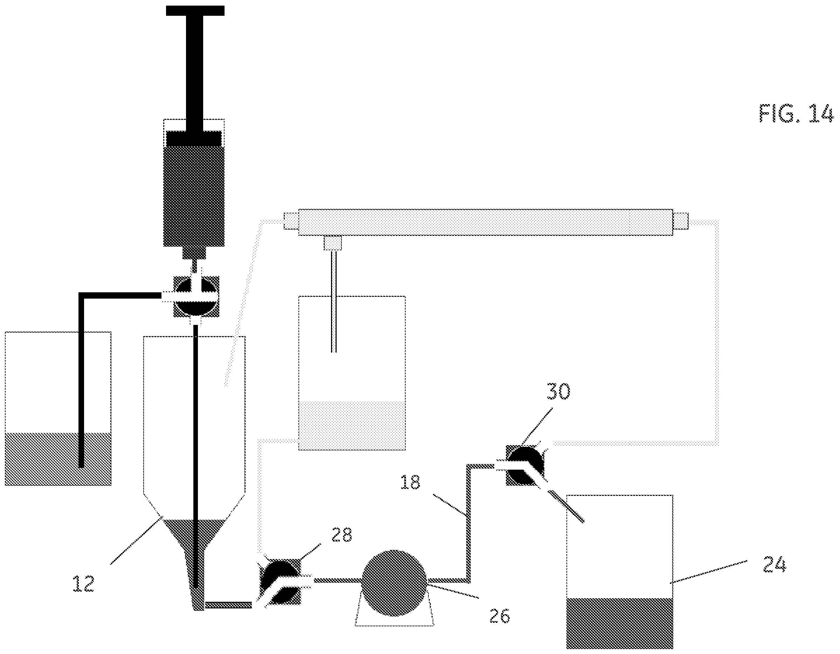

FIG. 14 is a schematic drawing of the embodiment shown in FIG. 13 showing the target retentate being collected in the target retentate receptacle.

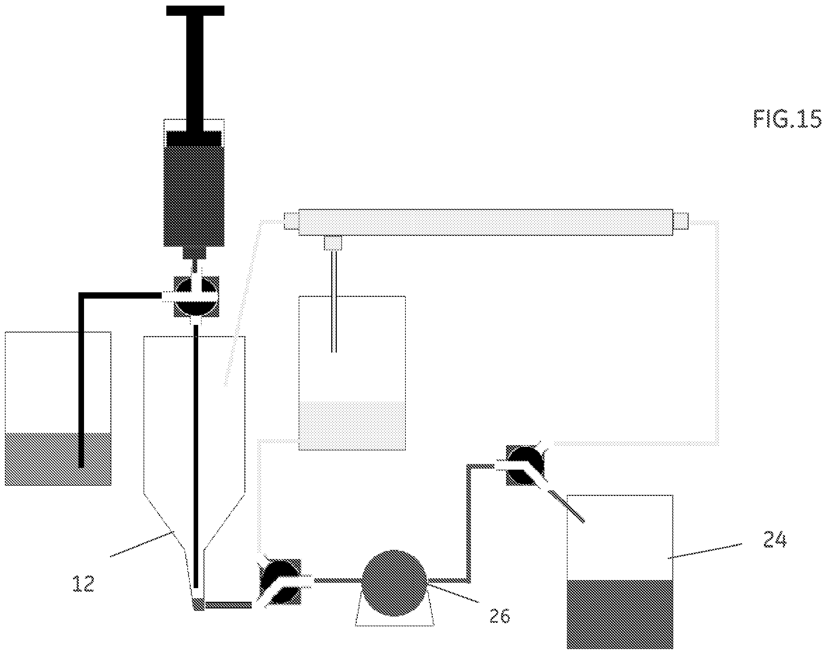

FIG. 15 is a schematic drawing of the embodiment shown in FIG. 14 showing the remaining amount of the target retentate at the bottom of vessel being transported to the target retentate receptacle.

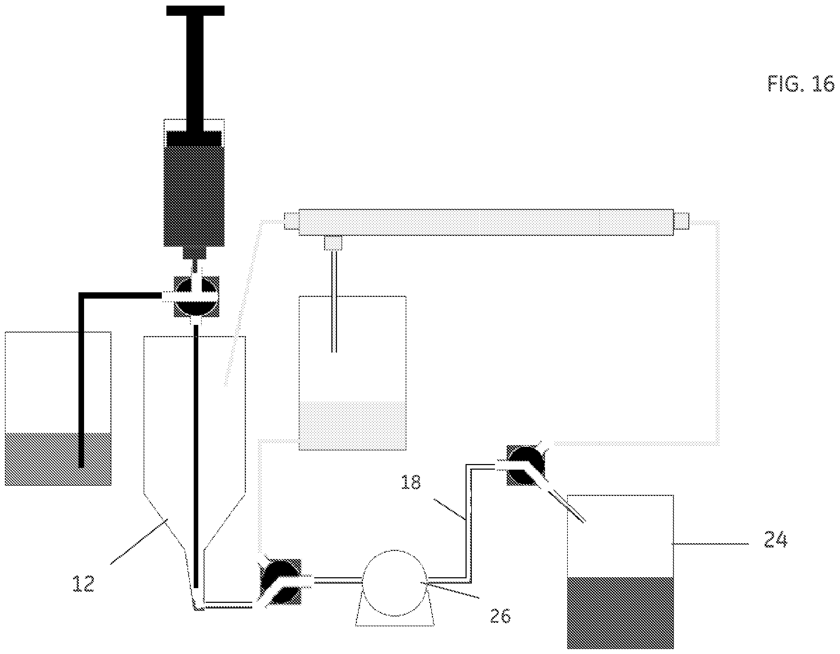

FIG. 16 is a schematic drawing of the embodiment shown in FIG. 15 showing a residual amount of target retentate in the conduit between the vessel land the target retentate receptacle.

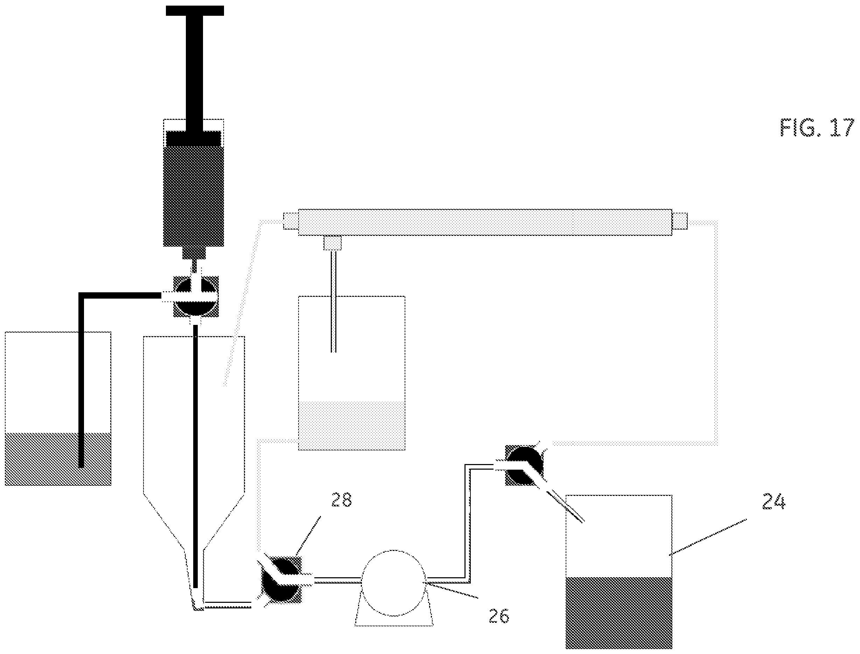

FIG. 17 is a schematic drawing of the embodiment shown in FIG. 16 showing the pump inlet value in an open position relative to the waste filtrate receptacle.

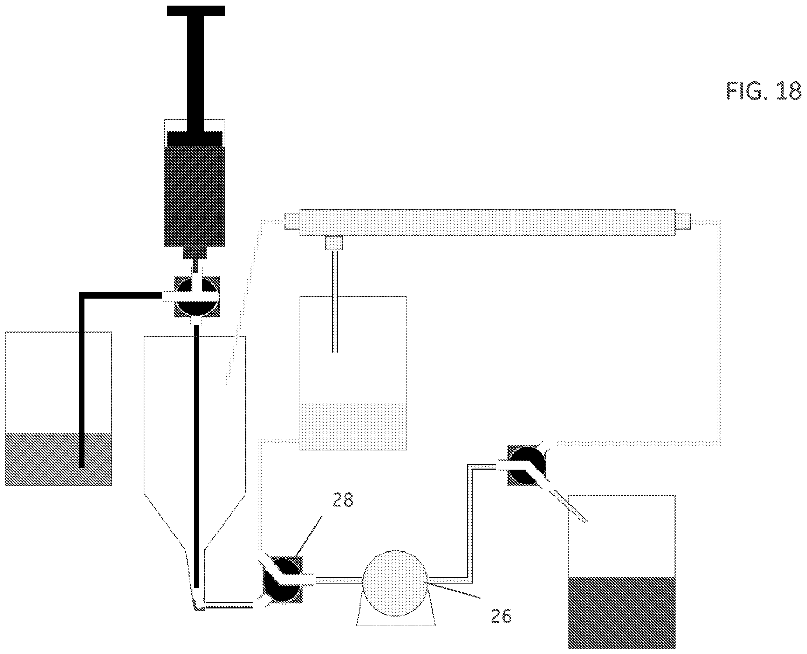

FIG. 18 is a schematic drawing of the embodiment shown in FIG. 17 showing the waste filtrate being transported through the conduit until the waste filtrate has pushed the residual target retentate in the conduit into the target retentate receptacle.

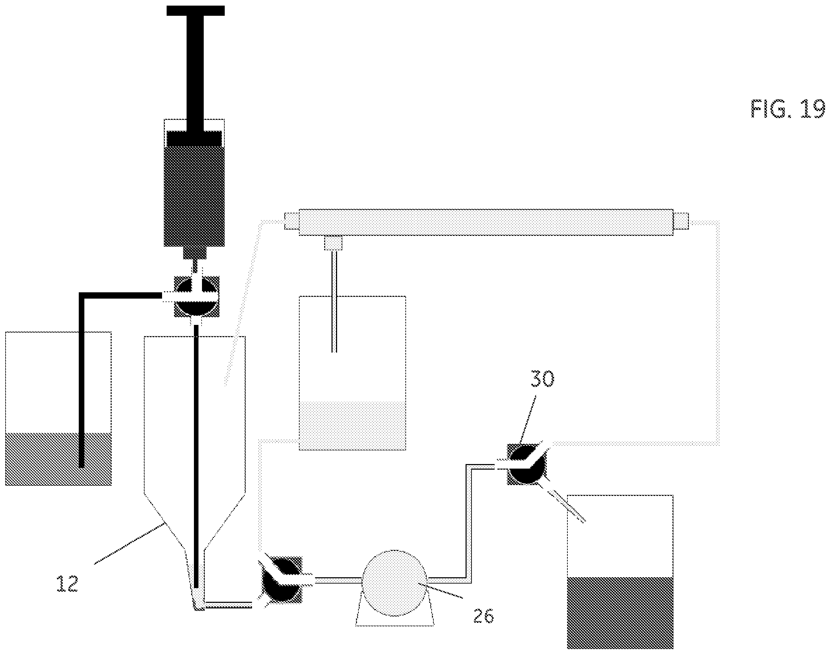

FIG. 19 is a schematic drawing of the embodiment shown in FIG. 18 showing the pump outlet value in a closed position relative to the target retentate receptacle and the waste filtrate being transported to the vessel.

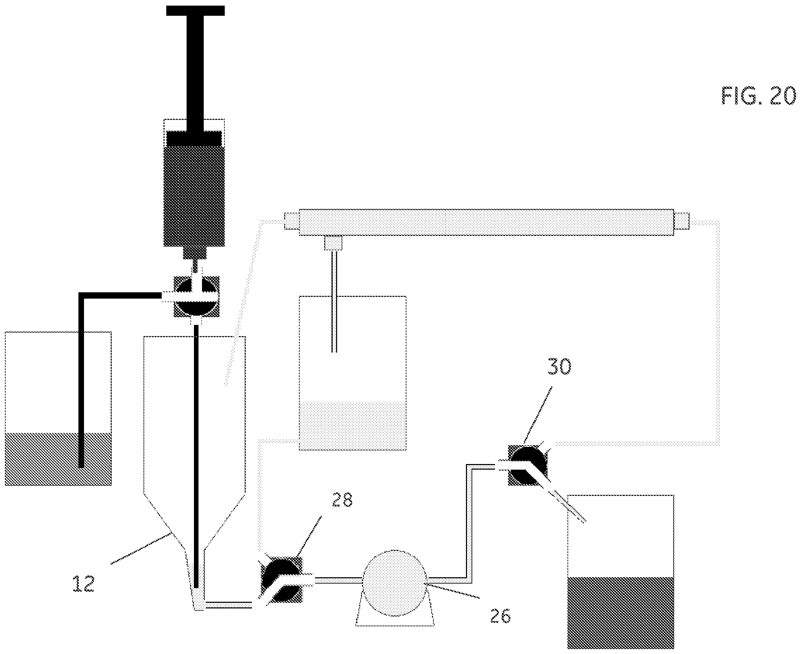

FIG. 20 is a schematic drawing of the embodiment shown in FIG. 19 showing the waste filtrate and any remaining target retentate in the system being collected in an auxiliary filtrate receptacle.

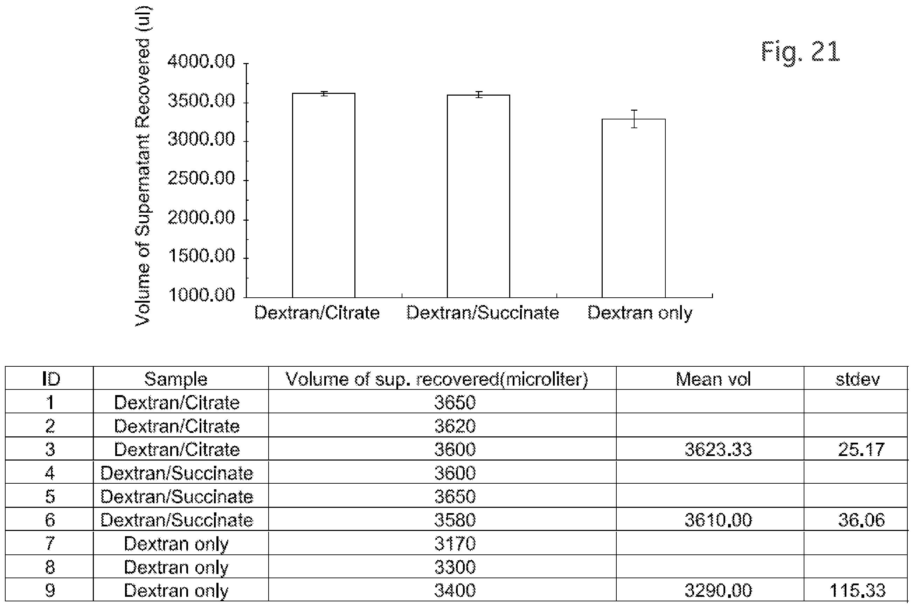

FIG. 21 is a graph and table showing examples of the volume of recovered TNC for dextran alone, dextran combined with sodium citrate and dextran combined with sodium succinate.

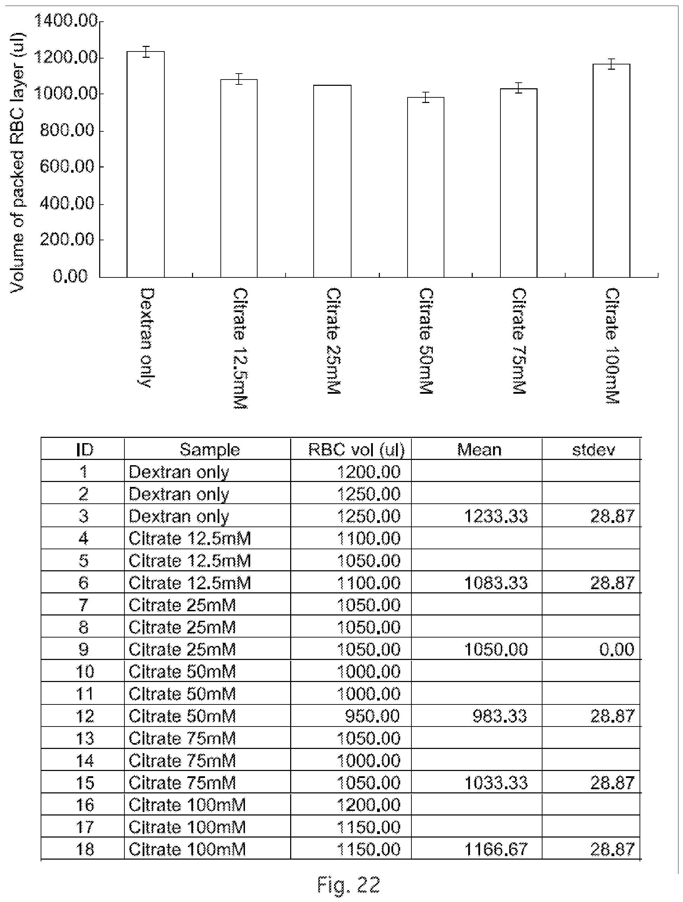

FIG. 22 is a graph showing an example of the sedimentation efficiency of sodium citrate.

### DETAILED DESCRIPTION

To more clearly and concisely describe and point out the subject matter of the claimed invention, the following definitions are provided for specific terms, which are used in the following description and the appended claims. Throughout the specification, exemplification of specific terms should be considered as non-limiting examples.

As used herein, the term "vessel" refers to any object capable of containing a liquid within its confines for at least a temporary period of time having at least one port.

As used herein, the term "biological material" refers to any material of a biological nature that can be aggregated into two or more submaterials. Non-limiting examples of biological materials are whole blood, cord blood and bone marrow that can be separated via aggregation and sedimentation/removal of RBCs while nucleated cells remain in a plasma solution. Nucleated cells include WBCs and rare stem cells.

One embodiment of the methods for processing nucleated cells generally comprises the separation and enrichment of nucleated cells, such as, but not limited to, rare stem cells, from cell samples including, but not limited to, blood and bone marrow. The filtration-based embodiment comprises two general steps. The first step comprises contacting the cell sample with a settling solution, such as a red blood cell aggregating agent (e.g. Dextran) with or without the addition of an enhancing agent (e.g. sodium citrate, sodium succinate). The enhancing agent in this example embodiment is added to enhance the RBC sedimentation rate and/or reduce the final RBC packed volume following sedimentation. Subsequently the aggregated RBCs are removed from the upper fraction containing plasma and nucleated cells by drainage, drawing off or other suitable means of transfer. The second step comprises volume reduction and nucleated cell concentration by filtering the RBC-depleted sample. One example of filtration uses a hollow-fiber filtration cartridge (General Electric Healthcare, Piscataway, NJ). This embodiment provides high cell recoveries (e.g. minimal cell trapping), minimal cell damage, and fast processing times. This example of the method is adaptable for use in the automated closed-system system. The methods and systems are adaptable for sterile processing of complex biological materials such as but not limited to cord blood and other cell sample materials.

### Example

The two-step automated example methods, that combine separation followed by filtration, rather than mere filtration, centrifugation, or magnetic separation alone, provide (1) increased total nucleated cell (TNC) recovery, (2) increased RBC removal, and (3) greater flexibility in handing a range of sample volumes (e.g. 50 to 300mL blood) than centrifugation due to the fixed physical dimensions of the centrifuge's sample holder. Unlike a centrifuge with a fixed sized, the filters used in the systems and methods may be scaled according to the sample volume.

The volume of starting material is determined (e.g. by weight, visual inspection). The required amount of RBC aggregating reagents is calculated based on the desired stoichiometric ratio (typically 1:1 or 1:2, blood to Dextran).

The cell sample starting material is transferred to a processing vessel. The RBC aggregating reagent(s) are also transferred to the processing vessel. The sample and reagents are then mixed and allowed to incubate ∼ 20min for RBC aggregation and gravity sedimentation.

The aggregate RBC fraction is then extracted from the vessel from the upper white blood cell (WBC)/plasma fraction (e.g. by pumping, pipetting, or drainage). The remaining WBC/plasma fraction is then transferred (e.g. by pumping, positive or negative pressure) to a filtration device (e.g. hollow fiber cartridge) having a suitable pore size (e.g. approximately 0.65 um pores). Excess plasma passes through the filtration device and is collected in a waste filtrate receptacle, while WBCs are retained.

The fraction sample is recirculated through the filtration device until the sample volume is concentrated to the desired final volume (e.g. 5-20 ml). The sample is transferred to a target retentate receptacle, typically for longterm cryo-storage. The filter and tubing is then purged to recover cells present in this "dead volume", typically using plasma and/or air. This material is then added to the concentrated sample. High total nucleated cell recovery (>85%) and viability (>95%) are achieved.

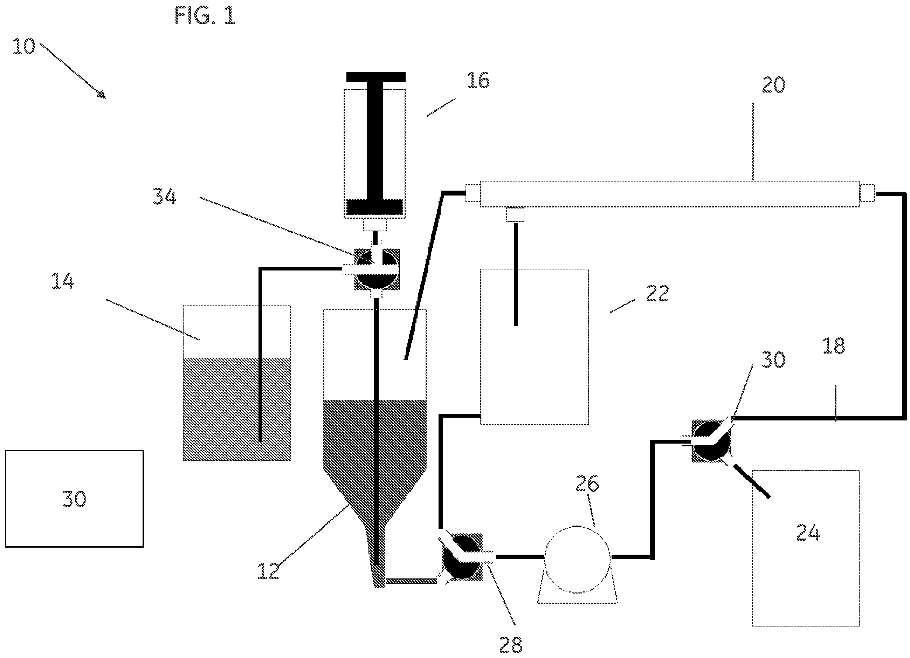

FIG. 1 is a schematic drawing of an embodiment of the system showing a biological sample and an enhancing agent in a mixing vessel and an agent receptacle, respectively. The embodiment of the system shown and generally referred to in FIG. 1 as system 10 comprises vessel 12 for containing and enabling the biological material to separate into two or more distinct submaterials; extraction device 16 for removing at least one of the submaterials from the vessel; filtration device 20; conduit 18 that transports one or more submaterials between the vessel and the filtration device; and control device 30 for controlling at least the transporting of one or more of the submaterials between the vessel and the filtration device via the conduit. This embodiment is only an example configuration of the system. The number and type of components can be varied as needed for a given set up and the order and flow of materials through the system may be varied as well as needed. For example, the materials may be extracted, stored, flushed and mixed using various configurations and components.

In the embodiment shown in FIG. 1, vessel 12 has an opening at the top through which the extracting device, which in this example is a syringe that is in fluid communication with valve 34, introduces the agents and withdraws materials and submaterials from vessel 12 at various times during the process.

System 10 also comprises receptacles for at least temporarily storing one or more filtrates. One of the receptacles in this embodiment is waste filtrate receptacle 22 and target retentate receptacle 24. System 10 further comprises valve 30 along the conduit for selectively directing target retentate into the target retentate receptacle; valve 28 along the conduit for selectively recirculating the waste filtrate at least partially through the conduit, and valve 34 for selectively introducing one or more agents into vessel 12, extracting materials from vessel 12 to mix the agents with the sample, and extracting one or more of the submaterials from vessel 12 after aggregation of the submaterials.

System 10 may also comprise one or more sensors such as sensor 32. Sensor 32 may be used to sensing one or more parameters of the materials in vessel 12 including but not limited to the presence of a submaterial at a given location within the vessel; the environmental conditions within the vessel such as but not limited to, temperature, pH, humidity, and pressure; and qualities or characteristics of the biological materials or submaterials. The sensors may be, but are not limited to, optical sensors, ultrasonic sensors, piezoelectric sensors, motion sensors, RFID sensors, electromagnetic sensors and load sensors.

System 10 also comprises a control subsystem 30 (also referred to herein as a controller) for automating and coordinating pump 26, extraction device 16 and valves 28, 34 and 30. Control subsystem 30 may also be configured to receive input from the user of the system and automatically determine the amount and/or type of agents to be added to vessel 12 based on the amount and type of materials introduced into vessel 12 to be process using the system. The system may be fully or partially automated by the control subsystem depending on the configuration of the a given system. The agents may be contained within a removable cassette that is inserted into a port in the system as needed depending on the type or amount of materials and submaterials to be processed.

System 10 further comprises pump 26, in fluid communication with the conduit, to facilitating the transport of one or more submaterials between the various components of the system. Pump 26 in this embodiment is a peristaltic pump but may comprise any type of pump suited to the configuration of the system.

Vessel 12 of the system may be adapted to separate the material into aggregated submaterials at least in part based on the relative weight of two or more submaterials. The submaterials separate into sedimentary layers and the extraction device in this embodiment is adapted to draw off or otherwise extract one or more of the sedimentary layers. In the embodiment shown in FIG. 1, extraction device 16 comprises a pick up line with a distal end located towards the bottom of vessel 12 to draw off a lowermost layer within the vessel once the submaterials have separated into their respective sedimentary layers. The extraction device may alternatively, or additionally, draw off an uppermost layer within the vessel, or one or more layers in between the lowermost and uppermost, depending on the configuration of the extraction device relative to the vessel.

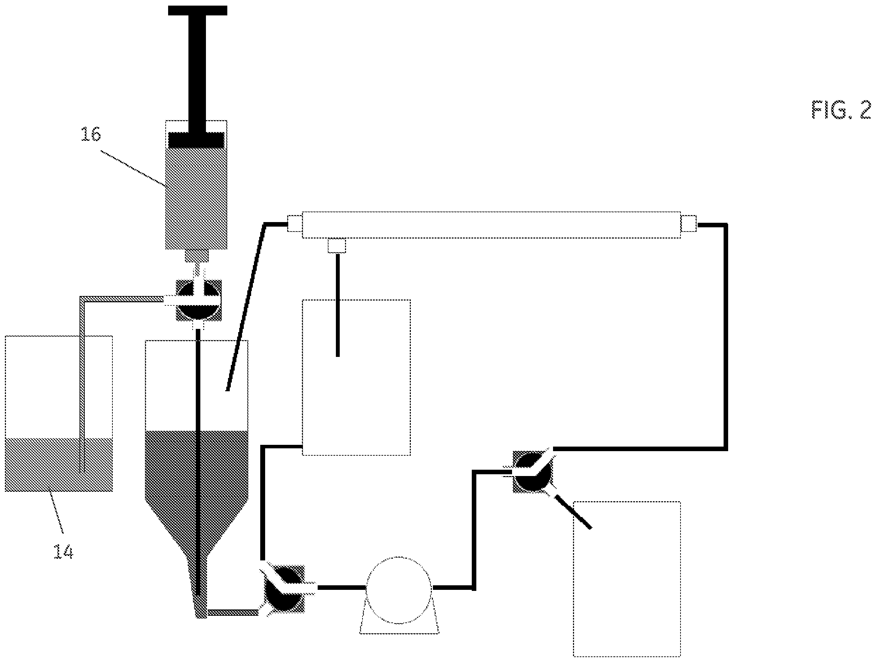

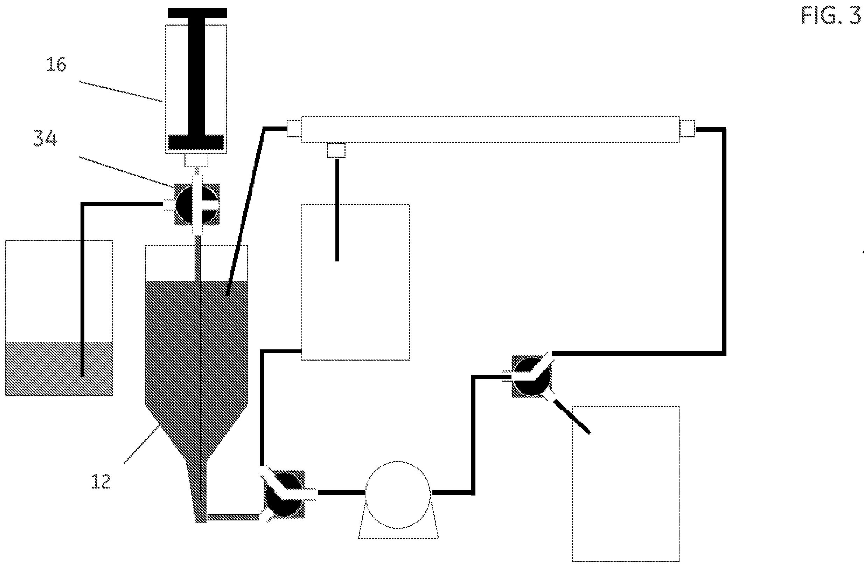

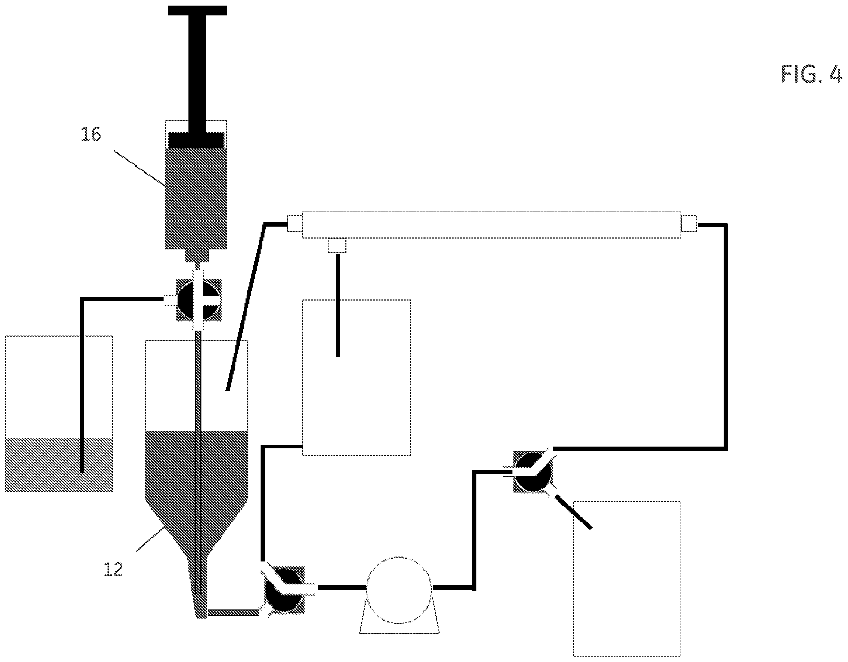

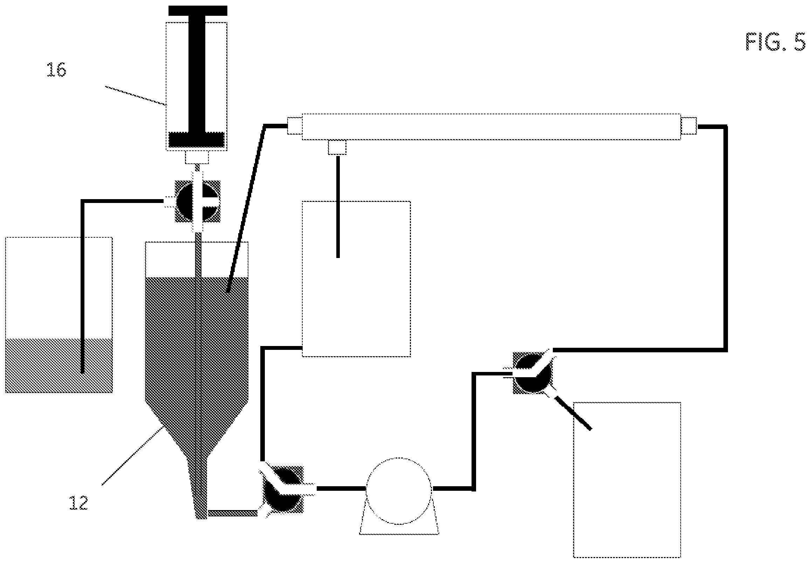

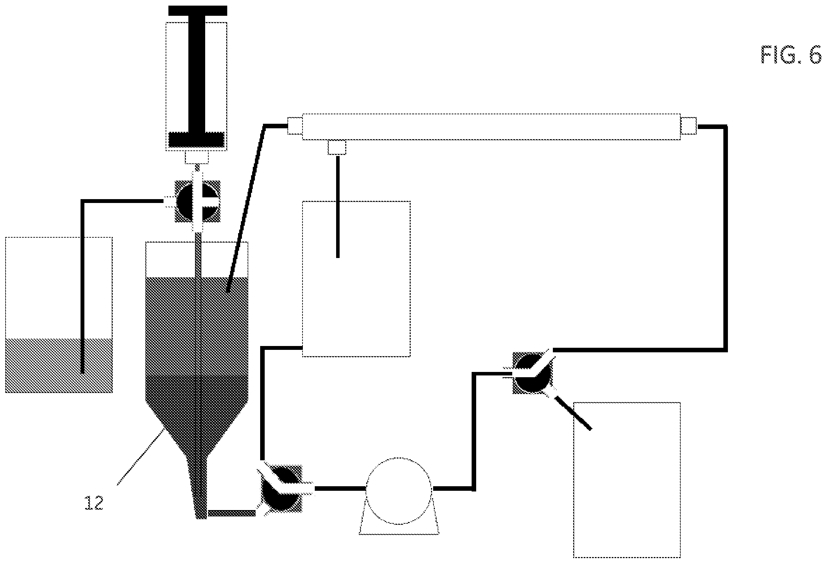

Syringe 16 together with valve 34, in fluid communication with agent receptacle 14, selectively remove a determined amount of agent from the agent receptacle and introduce the determined amount of agent into vessel 12 as shown in FIGs. 2 and 3. The extracting device in this example may be further adapted to draw a determined amount of material from the vessel, into which the agent has previously been introduced, into the extracting device and then return the drawn material back into the vessel, to facilitate mixing of the material with the agent as shown in FIGs. 4 and 5. To carry out the mixing step, valve 34 closes relative to receptacle 14 and opens relative to vessel 12 open the fluid communication between syringe 16 and vessel 12. Once the aggregating agents are mixed with the materials (e.g. whole blood) in vessel 12, the mixture typically needs time to settle into its various sedimentary layers. For whole blood or cord blood mixed, for example, with Dextran and sodium citrate, settling should occur within 20 minutes as shown in FIG. 6.

A sensing device such as sensor 32 may be used for determine when the submaterials have aggregated and separated into their respective layers by determining the location or level of at least one of the submaterials in the vessel.

Possible agents, for use in this example in which whole blood is being processed, are dextran (an aggregant), and sodium citrate and sodium succinate, which both enhance aggregation. These three examples of agents enhance the methods and systems by acting as aggregating agents and/or aggregation enhancing agents to initiate and accelerate the aggregation and sedimentation of the different types of submaterials, such as WBCs and RBCs, in the biological sample.

## Claims

1. A method for processing whole blood, cord blood or bone marrow comprising,
providing a biological material in a vessel;
adding an aggregating agent and an aggregation enhancing agent selected from citrate, succinate, a salt thereof, or a combination thereof to the material in the vessel and allowing the material to separate into two or more distinct submaterials;
extracting one or more of the submaterials from the vessel;
automatically transporting one or more of the submaterials remaining in the vessel to a filtration device; and
collecting a resulting target retentate comprising nucleated cells into a target retentate receptacle.

2. The method of claim 1, further comprising sensing the presence of one or more of the WBCs and RBCs in the vessel.

3. The method of any one of claims 1 or 2, wherein one or more of the WBCs and RBCs are extracted until a predetermined set point is reached.

4. The method of any one of claims 1-3, further comprising directing a resulting waste filtrate into a waste filtrate receptacle.

5. The method of claim 4, further comprising flushing the filtration device with the waste filtrate after the target retentate is collected in the target retentate receptacle.

6. The method of any one of claims 1-5, wherein the target retentate comprises nucleated cells, such as stem cells.

7. The method of one or more of the above claims, wherein the enhancing agent has a final concentration range from 10mM to 100mM, such as from 12.5mM to 75mM, from 25mM to 75mM or from 50mM to 75mM.

## Patentansprüche

1. Verfahren zur Verarbeitung von Vollblut, Nabelschnurblut oder Knochenmark, umfassend
Bereitstellen eines biologischen Materials in einem Gefäß;
Hinzufügen eines Aggregiermittels und eines Aggretationsverstärkungsmittels, die aus Citrat, Succinat, einem Salz davon oder einer Kombination davon gewählt sind, zu dem Material in dem Gefäß, und Gestatten, dass sich das Material in zwei oder mehr verschiedene Submaterialien trennt;
Extrahieren eines oder mehrerer der Submaterialien aus dem Gefäß;
automatisches Transportieren eines oder mehrerer der in dem Gefäß verbliebenen Submaterialen zu einer Filtrationsvorrichtung; und
Sammeln eines sich ergebenden Zielretentats, das kernhaltige Zellen umfasst, in einem Zielretentataufnahmebehälter.

2. Verfahren nach Anspruch 1, ferner umfassend das Erfassen des Vorhandenseins eines oder mehrerer aus den weißen Blutkörperchen und den roten Blutkörperchen in dem Gefäß.

3. Verfahren nach einem der Ansprüche 1 oder 2, wobei eines oder mehrere aus den weißen Blutkörperchen und den roten Blutkörperchen extrahiert werden, bis ein vorherbestimmter Sollwert erreicht wird.

4. Verfahren nach einem der Ansprüche 1 bis 3, ferner umfassend das Leiten eines sich ergebenden Abfallfitrats in einen Abfallfiltrataufnahmebehälter.

5. Verfahren nach Anspruch 4, ferner umfassend das Spülen der Filtrationsvorrichtung mit dem Abfallfiltrat, nachdem das Zielretentat in dem Zielretentataufnahmebehälter gesammelt wurde.

6. Verfahren nach einem der Ansprüche 1 bis 5, wobei das Zielretentat kernhaltige Zellen wie etwa Stammzellen umfasst.

7. Verfahren nach einem oder mehreren der obigen Ansprüche, wobei das Verstärkungsmittel einen Endkonzentrationsbereich von 10 mM bis 100 mM wie etwa von 12,5 mM bis 75 mM, von 25 mM bis 75 mM oder von 50 mM bis 75 mM aufweist.

## Revendications

1. Procédé de traitement de sang entier, de sang cordonal ou de moëlle osseuse comprenant :
la fourniture d'une matière biologique dans un récipient ;
l'addition d'un agent d'agrégation et d'un agent promoteur d'agrégation choisi parmi un citrate, un succinate, un sel de celui-ci ou une combinaison de ceux-ci à la matière dans le récipient en permettant à la matière de se séparer en deux ou plusieurs sous-matières distinctes ;
l'extraction de d'une ou plusieurs des sous-matières du récipient ;
le transport automatique d'une ou plusieurs des sous-matières restant dans le récipient à un dispositif de filtration ; et
le recueil d'un rétentat cible obtenu comprenant des cellules nucléées dans un réceptacle de rétentat cible.

2. Procédé selon la revendication 1, comprenant en outre la détection de la présence d'un ou plusieurs des WBC et RBC dans le récipient.

3. Procédé selon l'une quelconque des revendications 1 ou 2, dans lequel un ou plusieurs des WBC et RBC sont extraits jusqu'à ce qu'un point de consigne prédéterminé soit atteint.

4. Procédé selon l'une quelconque des revendications 1 à 3, comprenant en outre l'acheminement d'un filtrat de déchets obtenu dans un réceptacle à filtrat de déchets.

5. Procédé selon la revendication 4, comprenant en outre la purge du dispositif de filtration avec le filtrat de déchets une fois que le rétentat cible est recueilli dans le réceptacle à rétentat cible.

6. Procédé selon l'une quelconque des revendications 1 à 5, dans lequel le rétentat cible comprend des cellules nucléées, telles que des cellules souches.

7. Procédé selon une ou plusieurs des revendications précédentes, dans lequel l'agent promoteur a une plage de concentration finale de 10 mM à 100 mM, notamment de 12,5 mM à 75 mM, de 25 mM à 75 mM ou de 50 mM à 75 mM.
